(19) Europäisches Patentamt
European Patent Office
Office européen des brevets

(11) Veröffentlichungsnummer: **0 424 777 B1**

(12) # EUROPÄISCHE PATENTSCHRIFT

(45) Veröffentlichungstag der Patentschrift: **14.09.94**

(51) Int. Cl.$^5$: **C09B 57/00**, G11B 7/24, C07C 69/007, C07D 307/68

(21) Anmeldenummer: **90119730.1**

(22) Anmeldetag: **15.10.90**

Die Akte enthält technische Angaben, die nach dem Eingang der Anmeldung eingereicht wurden und die nicht in dieser Patentschrift enthalten sind.

(54) **Estergruppen aufweisende Azulenquadratsäurefarbstoffe, deren Zwischenprodukte sowie optisches Aufzeichnungsmedium.**

(30) Priorität: **25.10.89 DE 3935524**

(43) Veröffentlichungstag der Anmeldung:
**02.05.91 Patentblatt 91/18**

(45) Bekanntmachung des Hinweises auf die Patenterteilung:
**14.09.94 Patentblatt 94/37**

(84) Benannte Vertragsstaaten:
**CH DE FR GB IT LI**

(56) Entgegenhaltungen:
**EP-A- 0 310 080**

**JOURNAL OF ORGANIC CHEMISTRY, Band 38, Nr. 6, 1973, Seiten 1106-1113, American-Chemical Society, Easton, US; R.N. McDONALD et al.: "Nonbenzenoid aromaticsystems"**

(73) Patentinhaber: **BASF Aktiengesellschaft**
**Carl-Bosch-Strasse 38**
**D-67063 Ludwigshafen (DE)**

(72) Erfinder: **Schmitt, Michael, Dr.**
**Freudenbergstrasse 18**
**W-6940 Weinheim (DE)**
Erfinder: **Schrott, Wolfgang, Dr.**
**Brüsseler Ring 45**
**W-6700 Ludwigshafen (DE)**
Erfinder: **Neumann, Peter, Dr.**
**Poststrasse 28**
**W-6800 Mannheim 31 (DE)**
Erfinder: **Brosius, Sibylle, Dr.**
**Cordovastrasse 29**
**W-6700 Ludwigshafen (DE)**
Erfinder: **Schomann, Klaus Dieter, Dr.**
**Kopernikusstrasse 47**
**W-6700 Ludwigshafen (DE)**
Erfinder: **Kuppelmaier, Harald, Dr.**
**In den Bannzäunen 17**
**W-6701 Goennheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue Estergruppen aufweisende Azulenquadratsäurefarbstoffe der Formel I

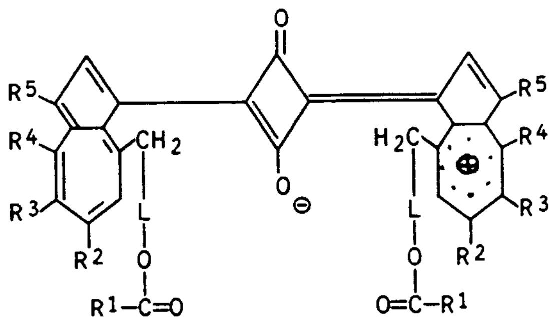

(I) ,

in der

| | |
|---|---|
| L | $C_1$-$C_{12}$-Alkylen, das gegebenenfalls durch Phenyl substituiert ist, |
| $R^1$ | einfach oder mehrfach ungesättigtes $C_2$-$C_{12}$-Alkenyl, das durch Phenyl substituiert sein kann, $C_5$-$C_7$-Cycloalkenyl, Pyrrolyl, Furanyl, Thienyl oder Pyridyl und |
| $R^2$, $R^3$, $R^4$ und $R^5$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxy-carbonyl oder Cyano substituiert ist, |

bedeuten, mit der Maßgabe, daß wenn $R^5$ Wasserstoff bedeutet, an einem oder beiden Azulenringen die Ringpositionen der Substituenten $CH_2$-L-O-CO-$R^1$ und $R^4$ innerhalb eines Azulenringes auch gegeneinander vertauscht sein können, deren Zwischenprodukte sowie ein optisches Aufzeichnungsmedium, das die neuen Farbstoffe enthält.

Zur kostengünstigen Herstellung optischer Datenaufzeichnungsträger werden Farbstoffe mit besonderen Eigenschaften benötigt. Diese Farbstoffe sollten

- eine starke Absorption zwischen 700 und 900 nm aufweisen, um mit Halbleiterlasern beschreibbare Schichten zu liefern,
- in Schicht eine hohe Reflektivität im nahen Infrarot (700 bis 900 nm) aufweisen, um mit einem einfachen Schichtaufbau (ohne Reflektorschicht) auszukommen,
- eine hohe Löslichkeit aufweisen, um beispielsweise die dünne Speicherschicht durch Spincoating auf einen Träger aufbringen zu können und
- in dünnen Schichten eine hohe Stabilität aufweisen.

Die bisher bekannten Speichermaterialien weisen häufig Zumindest in einem der genannten Anforderungspunkte Mängel auf.

Aufgabe der vorliegenden Erfindung war es daher, neue Farbstoffe bereitzustellen, bei denen die obengenannten Mängel nicht mehr oder höchstens nur in äußerst geringem Maße auftreten.

Demgemäß wurden die oben näher bezeichneten Estergruppen aufweisende Azulenquadratsäurefarbstoffe der Formel I gefunden.

Alle in der obengenannten Formel I auftretenden Alkylen- und Alkylgruppen können sowohl geradkettig als auch verzweigt sein.

Wenn in Formel I substituierte Phenylgruppen auftreten, können als Substituenten z.B. $C_1$-$C_4$-Alkyl, $C_1$-$C_4$-Alkoxy oder Halogen, dabei vorzugsweise Fluor, Chlor oder Brom, in Betracht kommen.

Reste L sind z.B. Methylen, Ethylen, 1,2- oder 1,3-Propylen, 1,2-, 1,3-, 2,3- oder 1,4-Butylen, Pentamethylen, Hexamethylen, Heptamethylen, Octamethylen, Nonamethylen, Decamethylen, Undecamethylen, Dodecamethylen, Phenylethylen, 1-Phenyl-1,3-propylen.

Reste $R^2$, $R^3$, $R^4$ und $R^5$ in Formel I sind beispielsweise Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Heptyl, Octyl, 2-Ethylhexyl, Nonyl, Decyl, Undecyl oder Dodecyl.

Reste $R^1$ sind z.B. Ethenyl, Propenyl, Butenyl, Butadienyl, Pentenyl, Pentadienyl, Hexenyl, Hexadienyl, Hexatrienyl, Heptenyl, Heptadienyl, Heptatrienyl, Octenyl, Octadienyl, Octatrienyl, Octatetraenyl, Nonenyl, Nonadienyl, Nonatrienyl, Nonatetraenyl, Decenyl, Decadienyl, Decatrienyl, Decatetraenyl, Decapentaenyl, Undecenyl, Undecadienyl, Undecatrienyl, Undecatetraenyl, Undecapentaenyl, Dodecenyl, Dodecadienyl, Dodecatrienyl, Dodecatetraenyl, Dodecapentaenyl, Dodecahexaenyl, Styryl, Cyclopentyl, Cyclopentenyl,

Cyclopentadienyl, Cyclohexyl, Cyclohexenyl, Cyclohexadienyl, Cycloheptyl, Cycloheptenyl, Cycloheptadienyl oder Cycloheptatrienyl.

Reste $R^2$, $R^3$, $R^4$ und $R^5$ sind weiterhin z.B. Fluormethyl, Chlormethyl, Difluormethyl, Trifluormethyl, Trichlormethyl, 2-Fluorethyl, 2-Chlorethyl, 2-Bromethyl, 1,1,1-Trifluorethyl, Heptafluorpropyl, 4-Chlorbutyl, 5-Fluorpentyl, 6-Chlorhexyl, Cyanomethyl, 2-Cyanoethyl, 3-Cyanopropyl, 2-Cyanobutyl, 4-Cyanobutyl, 5-Cyanopentyl, 6-Cyanohexyl, 2-Methoxyethyl, 2-Ethoxyethyl, 2-Propoxyethyl, 2-Isopropoxyethyl, 2-Butoxyethyl, 2-Methoxypropyl, 2-Ethoxypropyl, 3-Ethoxypropyl, 4-Ethoxybutyl, 4-Isopropoxybutyl, 5-Ethoxypentyl, 6-Methoxyhexyl, Benzyl, 1-Phenylethyl, 2-Phenylethyl, 4-Chlorbenzyl, 4-Methoxybenzyl, 2-(4-Methylphenyl)-ethyl, Methoxycarbonylmethyl, Ethoxycarbonylmethyl, 2-Methoxycarbonylethyl, 2-Ethoxycarbonylethyl, 3-Methoxycarbonylpropyl, 3-Ethoxycarbonylpropyl, 4-Methoxycarbonylbutyl, 4-Ethoxycarbonylbutyl, 5-Methoxycarbonylpentyl, 5-Ethoxycarbonylpentyl, 6-Methoxycarbonylhexyl oder 6-Ethoxycarbonylhexyl.

Bevorzugt sind Azulenquadratsäurefarbstoffe der Formel I in der $R^2$, $R^3$, $R^4$ und $R^5$ jeweils $C_1$-$C_6$-Alkyl oder Wasserstoff bedeuten.

Weiterhin bevorzugt sind Azulenquadratsäurefarbstoffe der Formel I, in der $R^1$ $C_2$-$C_{12}$-Alkenyl bedeutet.

Besonders bevorzugt sind Azulenquadratsäurefarbstoffe der Formel I, in der $R^2$ und $R^4$ jeweils Methyl und $R^3$ und $R^5$ jeweils Wasserstoff bedeuten und L und $R^1$ jeweils die obengenannte Bedeutung besitzen. Diese Farbstoffe entsprechen der Formel Ia

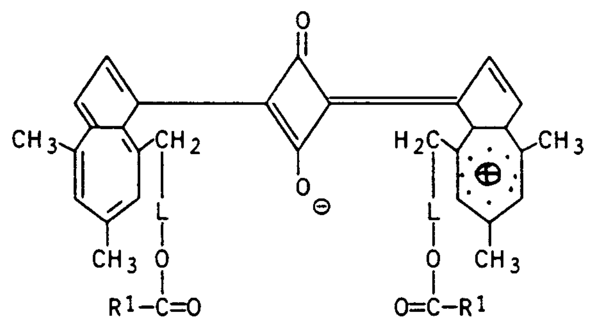

(Ia).

Ganz besonders bevorzugt sind Azulenquadratsäurefarbstoffe der Formel I, in der $R^2$ und $R^4$ jeweils Wasserstoff, $R^3$ Isopropyl und $R^5$ Methyl bedeuten und L und $R^1$ jeweils die obengenannte Bedeutung besitzen. Diese Farbstoffe entsprechen der Formel Ib

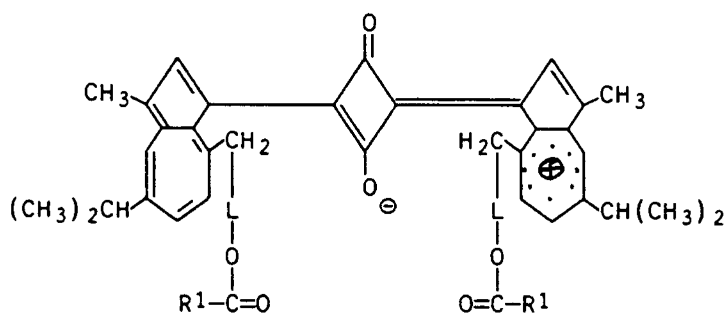

(Ib).

Die Farbstoffe der Formel I können z.B. aus Azulenderivaten der Formel II, in der L, $R^1$, $R^2$, $R^3$, $R^4$ und $R^5$ jeweils die obengenannte Bedeutung besitzen, durch Umsetzung mit Quadratsäure der Formel III gemäß der folgenden Gleichung erhalten werden:

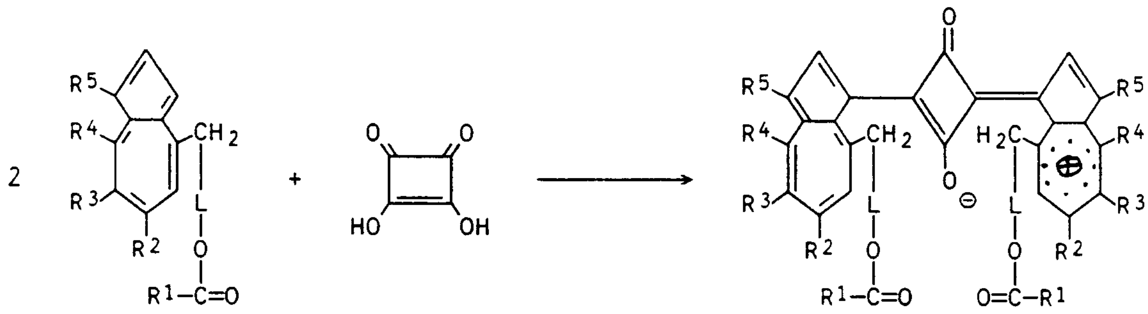

(II) (III) (I)

Bei denjenigen Azulenderivaten der Formel II, in denen $R^5$ Wasserstoff bedeutet, kann die Verknüpfung zur Quadratsäure an unterschiedlichen Ringpositionen des Fünfrings erfolgen, wobei isomere Produkte entstehen können, in denen die Ringpositionen der Substituenten $CH_2$-L-O-CO-$R^1$ und $R^4$, wie oben ausgeführt, gegeneinander vertauscht sind. Es sind nämlich dann Verbindungen, bei denen die Bindung zur Quadratsäure an derjenigen Seite erfolgt, an der der Substituent $CH_2$-L-O-CO-$R^1$ gebunden ist, von solchen zu unterscheiden, bei denen die Bindung zur Quadratsäure an derjenigen Seite erfolgt, an der der Substituent $R^5$ gebunden ist. Diese isomeren Verbindungen können chromatographisch getrennt werden. Für die Anwendung in Speicherschichten werden jedoch üblicherweise die Isomerengemische eingesetzt.

Das Herstellverfahren ist an sich bekannt und beispielsweise in Angew. Chem. 78, 937 (1966), oder in der EP-A-310 080 beschrieben.

Die EP-A-310 080 beschreibt Azulenquadratsäurefarbstoffe und Azulenderivate als deren Zwischenprodukte, die denen der Formel I und II ähnlich sind. Es werden dort z.B. Verbindungen aufgeführt, in denen $R^1$ Alkyl- oder Phenylreste darstellen.

Die Erfindung betrifft weiterhin neue Estergruppen aufweisende Azulene der Formel II

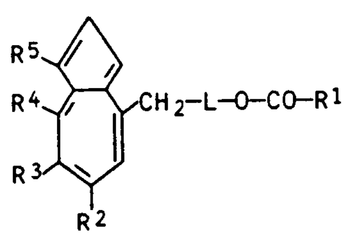

(II) ,

in der

| | |
|---|---|
| L | $C_1$-$C_{12}$-Alkylen, das gegebenenfalls durch Phenyl substituiert ist, |
| $R^1$ | einfach oder mehrfach ungesättigtes $C_2$-$C_{12}$-Alkenyl, das durch Phenyl substituiert sein kann, $C_5$-$C_7$-Cycloalkenyl, Pyrrolyl, Furanyl, Thienyl oder Pyridyl und |
| $R^2$, $R^3$, $R^4$ und $R^5$ | gleich oder verschieden sind und jeweils unabhängig voneinander Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, |

bedeuten.

Bezüglich der beispielhaften veranschaulichung der Reste L, $R^1$, $R^2$, $R^3$, $R^4$ und $R^5$ sei auf die voranstehend vorgenommene Aufzählung verwiesen.

Estergruppen aufweisende Azulenderivate der Formel II erhält man, wenn man von den entsprechenden Hydroxyalkylazulenderivaten der Formel (IV)

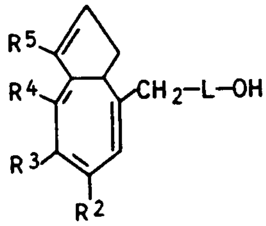

(IV) ,

in der L, $R^2$, $R^3$, $R^4$ und $R^5$ jeweils die obengenannte Bedeutung besitzen, ausgeht. Die Herstellung dieser Produkte ist beispielsweise in der EP-A-310 080 beschrieben

So eignen sich z.B. diejenigen Azulenderivate der Formel IVa oder IVb

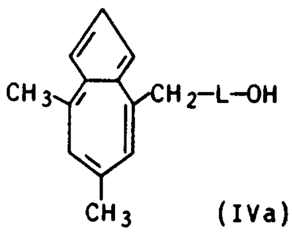

(IVa)

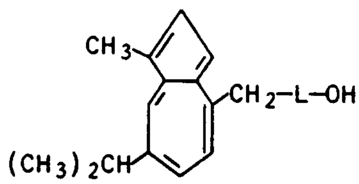

(IVb)

,

wobei L jeweils die obengenannte Bedeutung besitzt, besonders gut zur Umsetzung.

Die Umsetzung der Hydroxyalkylenderivate IV kann z.B. mit organischen Carbonsäuren oder Carbonsäurehalogeniden der Formel V

$R^1$-CO-X (V),

worin $R^1$ die obengenannte Bedeutung besitzt und X für Hydroxy oder Halogen steht, nach an sich bekannten Methoden erfolgen. Hierzu kann z.B. das Hydroxyalkylazulen IV mit einem Carbonsäurechlorid V (X = Chlor) in einem inerten organischen Lösungsmittel (z.B. Methylenchlorid, 1,1,2-Trichlorethan, Toluol, Ligroin oder Tetrahydrofuran) bei einer Temperatur von 0 bis 80 °C, gegebenenfalls in Gegenwart einer Base (z.B. tertiäre Amine oder Pyridin) umgesetzt werden. Weiterhin kann die Umsetzung des Hydroxyalkylazulens IV auch mit Carbonsärnren V (X = Hydroxy) in Gegenwart eines Kondensationsmittels, z.B. Dicyclohexylcarboiimid, gegebenenfalls in Anwesenheit eines Katalysators (z.B. Dimethylaminopyridin) unter den zuvor genannten Bedingungen erfolgen.

Eine weitere Aufgabe der vorliegenden Erfindung war es, ein neues optisches Aufzeichnungsmedium mit Azulenquadratsäurederivaten als Speichermaterialien bereitzustellen, das in einfacher Weise hergestellt werden kann, das gut beschreibbar und anschließend auch gut lesbar ist, wobei das Signal:Rausch-Verhältnis möglichst hoch sein sollte, und das eine hohe Stabilität der Speicherschichten aufweist.

Die Erfindung betrifft daher weiterhin ein optisches Aufzeichnungsmedium, enthaltend einen Träger sowie einen strahlungsempfindlichen, Farbstoff und gegebenenfalls Bindemittel enthaltenden dünnen Beschichtungsfilm, wobei der Farbstoff die Formel I

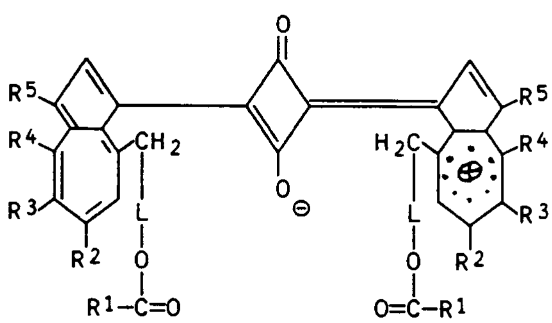

(I)

aufweist, in der

| | |
|---|---|
| L | $C_1$-$C_{12}$-Alkylen, das gegebenenfalls durch Phenyl substituiert ist, |
| $R^1$ | einfach oder mehrfach ungesättigtes $C_2$-$C_{12}$-Alkenyl, das durch Phenyl substituiert sein kann, $C_5$-$C_7$-Cycloalkenyl, Pyrrolyl, Furanyl, Thienyl oder Pyridyl und |
| $R^2$, $R^3$, $R^4$ und $R^5$ | gleich oder verschieden sind und jeweils unabhängig voneinander Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, |

bedeuten, mit der Maßgabe, daß wenn $R^5$ Wasserstoff bedeutet, an einem oder beiden Azulenringen die Ringpositionen der Substituenten $CH_2$-L-O-CO-$R^1$ und $R^4$ innerhalb eines Azulenringes auch gegeneinander vertauscht sein können.

Bevorzugt ist ein optisches Aufzeichnungsmedium, das Azulenquadratsäurefarbstoffe der Formel I enthält, in der $R^2$, $R^3$, $R^4$ und $R^5$ jeweils $C_1$-$C_6$-Alkyl oder Wasserstoff bedeuten.

Weiterhin bevorzugt ist ein optisches Aufzeichnungsmedium, das Azulenquadratsäurefarbstoffe der Formel I enthält, in der $R^1$ $C_2$-$C_{12}$-Alkenyl bedeutet.

Besonders bevorzugt ist ein optisches Aufzeichnungsmedium, das Azulenquadratsäurefarbstoffe der Formel I enthält, in der $R^2$ und $R^4$ jeweils Methyl und $R^3$ und $R^5$ jeweils Wasserstoff bedeuten.

Ganz besonders bevorzugt ist ein optisches Aufzeichnungsmedium, das Azulenquadratsäurefarbstoffe der Formel I enthält, in der $R^2$ und $R^4$ jeweils Wasserstoff, $R^3$ Isopropyl und $R^5$ Methyl bedeuten.

Als Träger kommen zweckmäßig transparente Träger, wie Glas oder Kunststoffe in Betracht. Geeignete Kunststoffe sind beispielsweise Poly(meth)-acrylate, Polycarbonate, Polyester, Epoxide, Polyolefine (Z.B. Polymethylpenten), Polyamid, Polyvinylchlorid, Polystyrol oder Polyvinylester.

Ein bevorzugtes Aufzeichnungsmedium weist einen Träger aus Polycarbonat oder Poly(meth)acrylat, insbesondere aber Polycarbonat auf.

Weiterhin bevorzugt ist ein optisches Aufzeichnungsmedium, das 1 bis 30 Gew.-% bezogen auf Farbstoff, an Bindemittel enthält.

Die neuen Azulenquadratsäurefarbstoffe der Formel I zeigen gute optische Daten. Darüber hinaus sind bei den neuen Verbindungen die reinen Farbstoffschichten sehr stabil. Es wurde nämlich keine Rekristallisation der reinen Farbstoffschicht beobachtet und somit kann auf den Zusatz polymerer Bindemittel auch verzichtet werden. Außerdem ist auch die Lichtechtheit (Stabilität) deutlich größer als bei bekannten Methinfarbstoffe, so daß der Zusatz von Stabilisatoren zur Schichtrezeptur auf ein Minimum begrenzt werden kann. Von besonderem Vorteil ist auch die gute Löslichkeit der neuen Farbstoffe I in den meisten organischen Lösungsmitteln, so daß diese Farbstoffe direkt (ohne Schutzschicht) auf strukturierte Kunststoffsubstrate, insbesondere Polycarbonatsubstrate aufgeschleudert werden können.

Wie oben ausgeführt, enthält die aufzuschleudernde Lösung vorzugsweise ein Bindemittel, um eine gute Langzeitstabilität zu gewährleisten und vor allem die optimale Viskosität des Aufzeichnungsmediums der aufzuschleudernden Lösung einzustellen. Vorzugsweise enthält die Lösung dabei 1 bis 30 Gew.-%, bezogen auf den Gehalt an gelöstem Feststoff der aufzuschleudernden Lösung, eines Bindemittels.

Als Bindemittel kommen z.B. Polyorganosiloxane, Epoxide, Poly(meth)-acrylate, Polystyrolhomo- und copolymerisate, Polyvinylcarbazol, Polyvinylpyrrolidon, Polyimldazolcopolymere, Polyvinylestercopolymere, Polyvinylethercopolymere, Polyvinylidenchloridpolymere, Acrylnitrilcopolymere, Polyvinylchlorid oder dessen Copolymere, Celluloseacetat oder Nitrocellulose in Betracht.

Ein bevorzugtes Aufzeichnungsmedium weist ein Bindemittel auf Basis eines Vinylpyrrolidon-Vinylacetat-Copolymeren oder eines Polyvinylchlorid-Polyvinylether-Copolymeren auf.

Das erfindungsgemäße optische Aufzeichnungsmedium wird zweckmäßig durch Aufschleudern einer Lösung, enthaltend organisches Lösungsmittel, Azulenquadratsäurefarbstoff I und gegebenenfalls Bindemittel hergestellt. Zweckmäßig weist die aufzuschleudernde Lösung dabei einen Gehalt an gelöstem Feststoff von 1 bis 30 Gew.-%, bezogen auf die Lösung, auf.

Geeignete Lösungsmittel sind z.B. Propanol, Isopropanol, Butanol, Diacetonalkohol, Methylethylketon, Toluol, Bromoform, 1,1,2-Trichlorethan oder deren Mischungen.

Gegebenenfalls kann die Lösung noch bis zu 10 Gew.-%, bezogen auf den Gehalt an gelöstem Feststoff der aufzuschleudernden Lösung, an Additiven, z.B. Antioxidantien, Singulett-Sauerstoff-Quencher oder UV-Absorber, enthalten.

Bevorzugt enthält die aufzuschleudernde Lösung bis zu 5 Gew.-%, bezogen auf den Gehalt an gelöstem Feststoff der aufzuschleudernden Lösung, eine Mischung von mehreren Antioxidantien, Singulett-Sauerstoff-Quenchern und UV-Absorbern. Bei Anwendung von solchen Antioxidantien, die ebenfalls im nahen Infrarot absorbieren, beispielsweise Nickelthiolenkomplexe, wie sie z.B. in der DE-A-3 505 750, DE-A-3 505 751 oder in Dyes and Pigments, Bd. 8, S. 381-388 (1987), beschrieben sind, können vorzugsweise bis zu 10 Gew.-%, bezogen auf den Gehalt an gelöstem Feststoff der aufzuschleudernden Lösung, in der

Lösung enthalten sein.

Unter Aufschleudern ist dabei das Aufbringen der Lösung auf den in Rotation befindlichen Träger, der zweckmäßig eine runde Form aufweist, zu verstehen. Es ist aber auch möglich, die Lösung auf den zunächst ruhenden Träger aufzubringen und ihn anschließend in Rotation zu versetzen. Das Aufgeben auf den Träger erfolgt zweckmäßig mit einer Spritze oder Kapillaren oder mittels einer mechanischen Pumpe.

Die Rotation des Trägers erfolgt im allgemeinen mit einer Geschwindigkeit von 5 bis 7000 U/min, vorzugsweise 500 bis 5000 U/min, wobei das Aufschleudern der Lösung zweckmäßig bei geringerer Drehzahl (ca. 500 bis 2000 U/min) und das daran anschließende Trockenschleudern bei höherer Drehzahl (ca. 5000 bis 7000 U/min) vorgenommen wird. Die Schichtdicke der gegenüber Laserlicht empfindlichen Schicht beträgt 40 bis 160 nm, vorzugsweise 80 bis 120 m. Sie ist abhängig von der Drehzahl, von der Konzentration und Viskosität der aufzuschleudernden Lösung sowie der Temperatur.

Beim erfindungsgemäßen optischen Aufzeichnungsmedium liegt die gegenüber Laserlicht empfindliche Schicht in Form einer homogenen, dünnen, glatten Schicht vor, die eine hohe optische Qualität aufweist. So liegen die Reflektivitätswerte im allgemeinen in einem Bereich der größer als 12 % ist.

Das neue Aufzeichnungsmedium ist ferner bei der Wellenlänge der verwendeten Laserlichtquelle hinreichend empfindlich, d.h. bei Einstrahlung von Lichtpulsen von wenigen nJ Energiegehalt, die auf Brennpunktdurchmesser von $\leqq 1$ $\mu$m fokussiert sind, bilden sich Pits aus, wobei ein ausgezeichnetes Signal:Rausch-Verhalten erzielt wird.

Als Laserlichtquelle eignen sich wegen der geringen Größe des Bauelementes, seines geringen Energiebedarfs und der Möglichkeit der direkten Modulation der optischen Ausgangsleistung durch Modulation des elektrischen Antriebsstromes Festkörper-Injektionslaser, die im nahen Infrarot emittieren, vor allem der AlGaAs-Laser, der im Wellenlängenbereich zwischen etwa 750 und 900 nm arbeitet, besonders gut.

Die folgenden Beispiele sollen die Erfindung näher erläutern.

## Beispiele

### Beispiel 1 (nicht beansprucht)

Darstellung von 3-(7-Isopropyl-1-methylazulen-4-yl)butylacetat

In eine Lösung aus 12,1 g (0,05 mol) 3-(7-Isopropyl-1-methylazulen-4-yl)-butanol und 7,9 g (0,1 mol) Pyridin in 100 ml Dichlormethan wurden bei 0 °C 4,8 g (0,063 mol) Acetylchlorid in 20 ml Dichlormethan getropft und 1 Stunde bei Raumtemperatur gerührt. Der Ansatz wurde mit 30 ml 2N Salzsäure ausgeschüttelt, neutral gewaschen und über Natriumsulfat getrocknet. Der nach Abziehen des Lösungsmittels verbleibende Rückstand wurde chromatographiert (Kieselgel; Petrolether/Ethylacetat 9:1 v/v). Man erhielt 12,6 g (89 %) Acetat als blaues hochviskoses Öl.

Physikalische Daten:

IR (KBr): 2959, 2929 (CH); 1741 (C=O); 1555, 1463, 1436, 1387, 1365, 1241, 1047 $cm^{-1}$.-$^1$H-NMR ($CDCl_3$)-:$\delta$ = 1.38 (d, 6H); 2.05 (s, 3H); 2.16 (cm, 2H); 2.68 (s, 3H); 3.08 (cm, 1H); 2.22 (cm, 2H); 4.18 (cm, 2H); 6.95 (d, 1H); 7.30 (d, 1H); 7.40 (d, 1H); 7.64 (d, 1H), 8.18 (s, 1H) ppm.-$^{13}$C-NMR ($CDCl_3$):$\delta$ = 12.86; 20.85; 24.72 (2C); 30.17; 34.53; 38.28; 64.22; 112.29; 124.41; 125.46; 133.30; 135.13; 136.39; 136.59; 137.28; 140.04; 147.55; 170.90 ppm.- MS: m/e = 284.4 ($C_{19}H_{24}O_2^+$, 50 %).-Analog Beispiel 1 wurden die in Tabelle 1 aufgeführten Carbonsäureester hergestellt und zusätzlich durch IR-, $^1$H-NMR-, $^{13}$C-NMR-, MS-Spektren sowie GC charakterisiert.

Bei der Umsetzung von Hydroxyalkylazulenen mit Croton- oder Sorbinsäurederivaten in Gegenwart stärkerer Basen, z.B. Triethylamin, erfolgt, im Gegensatz zur oben stehenden Vorschrift, eine teilweise oder vollständige Isomerisierung der Doppelbindung (vgl. Beispiel 2).

### Beispiel 2

Darstellung von 3-Butencarbonsäure-3-(7-isobutyl-1-methylazulen-4-yl)-butylester

In eine Lösung von 7,26 g (0,03 mol) 3-(7-isobutyl-1-methylazulen-4-yl)-butanol und 30 ml Triethylamin in 100 ml Tetrahydrofuran wurden bei Raumtemperatur 10,0 g (0,07 mol) Crotonsäurechlorid getropft und 2 Stunden gerührt. Danach wurden 50 ml Wasser zugegeben und kräftig gerührt. Der Ansatz wurde mit Methyl-t-butylether ausgeschüttelt, über Natriumsulfat getrocknet und der nach Abziehen des Lösungsmittels verbleibende Rückstand an Kieselgel (Petrolether, Ethylacetat 9:1 v/v) chromatographiert. Man erhielt 8,2 g (88 %) nach GC einheitliches Produkt als blaues hochviskoses Öl.

Physikalische Daten:

IR (KBr): 2959, 2927 (CH); 1738 (C=O); 1464, 1423, 1388, 1327, 1256, 1173, 921 $cm^{-1}$.-$^{1}H$-NMR ($CDCl_3$)-:$\delta$=1.47 (d, 6H); 2.18 (cm, 2H); 2.65 (s, 3H); 3.08 (m, 3H); 3.20 (t, 2H); 4.18 (t, 2H); 5.18 (m, 2H); 5.94 (m, 1H); 6.95 (d, 1H); 7.28 (bs, 1H); 7.40 (d, 1H), 7.62 (bs, 1H), 8.18 (s, 1H) ppm.-$^{13}C$-NMR ($CDCl_3$):$\delta$=12.84; 24.71 (2C); 30.18; 34.50; 38.28; 39.23, 64.41; 112.33; 118.36; 124.39; 125.47; 130.49; 133.28; 135.10; 136.42; 136.60; 137.31; 140.04; 147.49; 171.29 ppm.- MS: m/e = 310.4 ($C_{21}H_{26}O_2^+$, 80 %).-

Tabelle 1

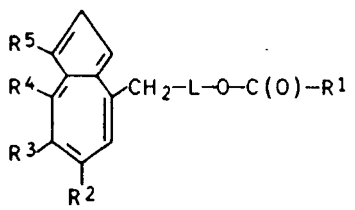

| Beispiel-Nr. | $-L-O-C(O)R^1$ | $R^2$ | $R^3$ | $R^4$ | $R^5$ | MS[M⊕] | IR (CO) [$cm^{-1}$] |
|---|---|---|---|---|---|---|---|
| 3 | $-CH_2-CH_2-O-C(O)-CH=CH_2$ | H | $C(CH_3)_2$<br>H | H | $CH_3$ | $C_{20}H_{24}O_2$<br>296.4 | 1724 |
| 4 | $-CH-CH_2-O-C(O)-CH=CH_2$<br>$C_2H_5$ | H | $C(CH_3)_2$<br>H | H | $CH_3$ | $C_{22}H_{28}O_2$<br>324.5 | 1726 |
| 5 | $CH_3$<br>$-CH_2-CH_2-O-C(O)-C=CH_2$ | H | $C(CH_3)_2$<br>H | H | $CH_3$ | $C_{21}H_{26}O_2$<br>310.4 | 1718 |
| 6 | $CH_3$<br>$-CH-CH_2-O-C(O)-C=CH_2$<br>$C_2H_5$ | H | $C(CH_3)_2$<br>H | H | $CH_3$ | $C_{23}H_{30}O_2$<br>338.5 | 1719 |
| 7 | $-CH_2-CH_2-O-C(O)-CH=CH-CH_3$ | H | $C(CH_3)_2$<br>H | H | $CH_3$ | $C_{21}H_{26}O_2$<br>310.4 | 1720 |
| 8 | $-CH-CH_2-O-C(O)-CH=CH-CH_3$<br>$C_2H_5$ | H | $C(CH_3)_2$<br>H | H | $CH_3$ | $C_{23}H_{30}O_2$<br>338.5 | 1721 |
| 9 | $-CH_2-CH_2-O-C(O)-CH=CH-CH=CH-CH_3$ | H | $C(CH_3)_2$<br>H | H | $CH_3$ | $C_{23}H_{28}O_2$<br>336.5 | 1713 |

Fortsetzung - Tabelle 1

| Beispiel-Nr. | $-L-O-C(O)R^1$ | $R^2$ | $R^3$ | $R^4$ | $R^5$ | MS[M⊕] | IR (CO) [$cm^{-1}$] |
|---|---|---|---|---|---|---|---|
| 10 | $-CH(C_2H_5)-CH_2-O-C(O)-CH=CH-CH=CH-CH_3$ | H | $C(CH_3)_2$<br>H | H | $CH_3$ | $C_{25}H_{32}O_2$<br>364.5 | 1713 |
| 11 | $-CH_2-CH_2-O-C(O)-CH=CH-$ 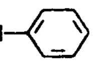 | H | $C(CH_3)_2$<br>H | H | $CH_3$ | $C_{26}H_{28}O_2$<br>372.5 | 1712 |
| 12 | $-CH(C_2H_5)-CH_2-O-C(O)-CH=CH-$ 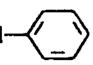 | H | $C(CH_3)_2$<br>H | H | $CH_3$ | $C_{28}H_{32}O_2$<br>400.6 | 1712 |
| 13 | $-CH_2-CH_2-O-C(O)$ 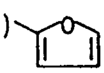 | H | $C(CH_3)_2$<br>H | H | $CH_3$ | $C_{22}H_{24}O_3$<br>336.4 | 1728 |
| 14 | $-CH(C_2H_5)-CH_2-O-C(O)$ 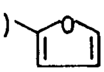 | H | $C(CH_3)_2$<br>H | H | $CH_3$ | $C_{24}H_{28}O_3$<br>364.5 | 1727 |

Beispiel 15 (nicht beansprucht)

Darstellung des Bis-[3-(7-Isobutyl-1-methylazulen-4-yl)butylacetat]-quadratsäurefarbstoffs

Eine Mischung aus 15,9 g (0,056 mol) 3-(7-Isobutyl-1-methylazulen-4-yl)-butylacetat, 5,0 g Quadratsäure und 80 ml Toluol/Butanol (1:1) wurde 2 Stunden am Wasserabscheider unter Rückfluß erhitzt. Nach Abdestillieren des Lösungsmittels wurde der Rückstand aus Ethylacetat umkristallisiert. Man erhielt 3,0 g (17 %) Farbstoff als hellbraune, metallisch glänzende Kristalle vom Schmp. 180 bis 181 °C.

Physikalische Daten:

IR (KBr): 2985 (CH); 1736, 1612 (C=O); 1437, 1386, 1337, 1323, 1252, 1243, 1020 $cm^{-1}$.- UV ($CH_2Cl_2$): $\lambda max(\epsilon)$ = 766 (116000) nm.- $^1H$-NMR ($CDCl_3$): $\delta$ = 1.38 (d, 12H); 1.89 (s, 6H); 1.95 (m, 4H); 2.55 (s, 6H); 3.08 (cm, 2H); 3.95 (m, 8H), 7.48 (d, 2H); 7.60 (d, 2H); 8.12 (s, 2H); 8.88 (s, 2H) ppm.- $^{13}C$-NMR ($CDCl_3$): $\delta$ = 12.96 (2C); 20.73 (4C); 24.21 (2C); 30.53 (2C); 36.17 (2C); 38.33 (2C); 63.66 (2C); 121.58 (2C); 130.87 (2C); 133.97 (2C); 134.38 (2C); 138.18 (2C); 139.82 (2C); 141.86 (2C); 147.49 (2C); 150.31 (2C); 155.54 (2C); 170.75 (2C); 181.79 (2C); 183.07 (2C) ppm.-MS: m/e = 646.8 ($C_{42}H_{46}O_6$, 50 %).-Analog Beispiel 24 wurden die in Tabelle 2 aufgeführten Quadratsäurefarbstoffe hergestellt und zusätzlich durch IR-, $^1H$-NMR-, $^{13}C$-NMR- und MS-Spektren charakterisiert.

Tabelle 2

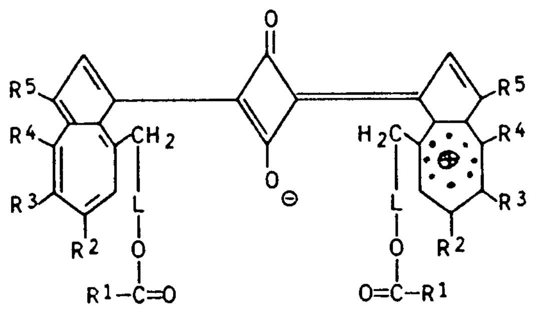

(I) ,

| Beispiel-Nr. | $-L-O-C(O)R^1$ | $R^2$ | $R^3$ | $R^4$ | $R^5$ | $\lambda_{max}(\varepsilon)$ $CH_2Cl_2$ | Schmp. [°C] |
|---|---|---|---|---|---|---|---|
| 16 | $-CH_2-CH_2-O-C(O)-CH=CH_2$ | H | $C(CH_3)_2$<br>H | H | $CH_3$ | 769<br>(112000) | 115-116 |
| 17 | $-CH-CH_2-O-C(O)-CH=CH_2$<br>$C_2H_5$ | H | $C(CH_3)_2$<br>H | H | $CH_3$ | 772<br>(107000) | 99-101 |
| 18 | $CH_3$<br>$-CH_2-CH_2-O-C(O)-C=CH_2$ | H | $C(CH_3)_2$<br>H | H | $CH_3$ | 770<br>(117000) | 135-136 |
| 19 | $CH_3$<br>$-CH-CH_2-O-C(O)-C=CH_2$<br>$C_2H_5$ | H | $C(CH_3)_2$<br>H | H | $CH_3$ | 773<br>(111000) | 171-172 |
| 20 | $-CH_2-CH_2-O-C(O)-CH=CH-CH_3$ | H | $C(CH_3)_2$<br>H | H | $CH_3$ | 770<br>(114000) | 183-184 |
| 21 | $-CH-CH_2-O-C(O)-CH=CH-CH_3$<br>$C_2H_5$ | H | $C(CH_3)_2$<br>H | H | $CH_3$ | 772<br>(106000) | 103-105 |

Fortsetzung - Tabelle 2

| Beispiel-Nr. | $-L-O-C(O)R^1$ | $R^2$ | $R^3$ | $R^4$ | $R^5$ | $\lambda_{max}(\varepsilon)$ $CH_2Cl_2$ | Schmp. [°C] |
|---|---|---|---|---|---|---|---|
| 22 | $-CH_2-CH_2-O-C(O)-CH_2-CH=CH_2$ | H | $C(CH_3)_2H$ | H | $CH_3$ | 770 (118000) | 97-98 |
| 23 | $-CH_2-CH_2-O-C(O)-CH=CH-CH=CH-CH_3$ | H | $C(CH_3)_2H$ | H | $CH_3$ | 770 (115000) | 129-130 |
| 24 | $-CH(C_2H_5)-CH_2-O-C(O)-CH=CH-CH=CH-CH_3$ | H | $C(CH_3)_2H$ | H | $CH_3$ | 772 (114000) | 213-214 |
| 25 | $-CH_2-CH_2-O-C(O)-CH=CH-$ 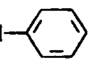 | H | $C(CH_3)_2H$ | H | $CH_3$ | 768 (117000) | 172-173 |
| 26 | $-CH(C_2H_5)-CH_2-O-C(O)-CH=CH-$ 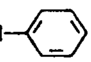 | H | $C(CH_3)_2H$ | H | $CH_3$ | 772 (118000) | 180-181 |
| 27 | $-CH_2-CH_2-O-C(O)$ 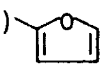 | H | $C(CH_3)_2H$ | H | $CH_3$ | 766 (118000) | 200-201 |
| 28 | $-CH(C_2H_5)-CH_2-O-C(O)$ 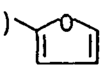 | H | $C(CH_3)_2H$ | H | $CH_3$ | 770 (108000) | 163-164 |

**Patentansprüche**

**1.** Estergruppen aufweisende Azulenquadratsäurefarbstoffe der Formel I

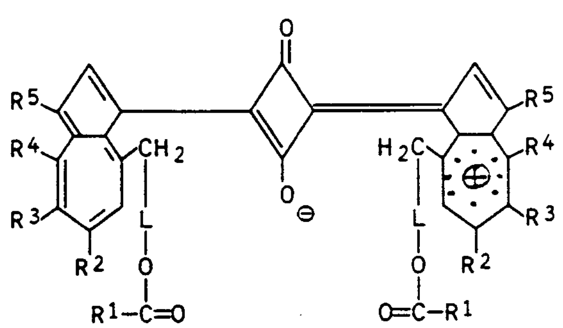

(I) ,

in der

| | |
|---|---|
| L | $C_1$-$C_{12}$-Alkylen, das gegebenenfalls durch Phenyl substituiert ist, |
| $R^1$ | einfach oder mehrfach ungesättigtes $C_2$-$C_{12}$-Alkenyl, das durch Phenyl substituiert sein kann, $C_5$-$C_7$-Cycloalkenyl, Pyrrolyl, Furanyl, Thienyl oder Pyridyl und |
| $R^2$, $R^3$, $R^4$ und $R^5$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, |

bedeuten, mit der Maßgabe, daß wenn $R^5$ Wasserstoff bedeutet, an einem oder beiden Azulenringen die Ringpositionen der Substituenten $CH_2$-L-O-CO-$R^1$ und $R^4$ innerhalb eines Azulenringes auch gegeneinander vertauscht sein können.

2. Azulenquadratsäurefarbstoffe nach Anspruch 1, dadurch gekennzeichnet, daß $R^2$, $R^3$, $R^4$ und $R^5$ jeweils $C_1$-$C_6$-Alkyl oder Wasserstoff bedeuten.

3. Azulenquadratsäurefarbstoffe nach Anspruch 1, dadurch gekennzeichnet, daß $R^2$ und $R^4$ jeweils Methyl und $R^3$ und $R^5$ jeweils Wasserstoff bedeuten.

4. Azulenquadratsäurefarbstoffe nach Anspruch 1, dadurch gekennzeichnet, daß $R^2$ und $R^4$ jeweils Wasserstoff, $R^3$ Isopropyl und $R^5$ Methyl bedeuten.

5. Optisches Aufzeichnungsmedium, enthaltend einen Träger sowie einen strahlungsempfindlichen, Farbstoff und gegebenenfalls Bindemittel enthaltenden dünnen Beschichtungsfilm, dadurch gekennzeichnet, daß der Farbstoff die Formel I

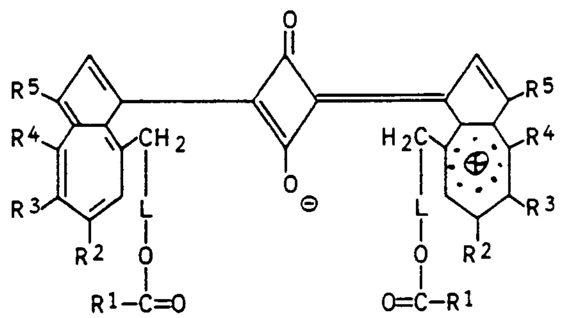

(I)

aufweist, in der

| | |
|---|---|
| L | $C_1$-$C_{12}$-Alkylen, das gegebenenfalls durch Phenyl substituiert ist, |
| $R^1$ | einfach oder mehrfach ungesättigtes $C_2$-$C_{12}$-Alkenyl, das durch Phenyl substituiert sein kann, $C_5$-$C_7$-Cycloalkenyl, Pyrrolyl, Furanyl, Thienyl oder Pyridyl und |
| $R^2$, $R^3$, $R^4$ und $R^5$ | gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist, |

bedeuten, mit der Maßgabe, daß wenn $R^5$ Wasserstoff bedeutet, an einem oder beiden Azulenringen die Ringpositionen der Substituenten $CH_2$-L-O-CO-$R^1$ und $R^4$ innerhalb eines Azulenringes auch gegeneinander vertauscht sein können.

6. Optisches Aufzeichnungsmedium nach Anspruch 5, dadurch gekennzeichnet, daß $R^2$, $R^3$, $R^4$ und $R^5$ jeweils $C_1$-$C_6$-Alkyl oder Wasserstoff bedeuten.

7. Optisches Aufzeichnungsmedium nach Anspruch 5, dadurch gekennzeichnet, daß $R^2$ und $R^4$ jeweils Methyl und $R^3$ und $R^5$ jeweils Wasserstoff bedeuten.

8. Optisches Aufzeichnungsmedium nach Anspruch 5, dadurch gekennzeichnet, daß $R^2$ und $R^4$ jeweils Wasserstoff, $R^3$ Isopropyl und $R^5$ Methyl bedeuten.

9. Estergruppen aufweisende Azulene der Formel II

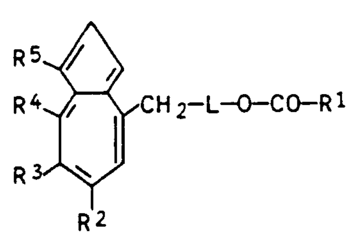

(II),

in der

L $C_1$-$C_{12}$-Alkylen, das gegebenenfalls durch Phenyl substituiert ist,

$R^1$ einfach oder mehrfach ungesättigtes $C_2$-$C_{12}$-Alkenyl, das durch Phenyl substituiert sein kann, $C_5$-$C_7$-Cycloalkenyl, Pyrrolyl, Furanyl, Thienyl oder Pyridyl,

$R^2$, $R^3$, $R^4$ und $R^5$ gleich oder verschieden sind und unabhängig voneinander jeweils Wasserstoff oder $C_1$-$C_{12}$-Alkyl, das gegebenenfalls durch Halogen, $C_1$-$C_{12}$-Alkoxy, Phenyl, substituiertes Phenyl, $C_1$-$C_{12}$-Alkoxycarbonyl oder Cyano substituiert ist,

bedeuten.

## Claims

1. A carbonyloxy-substituted azulenesquaric acid dye of the formula I

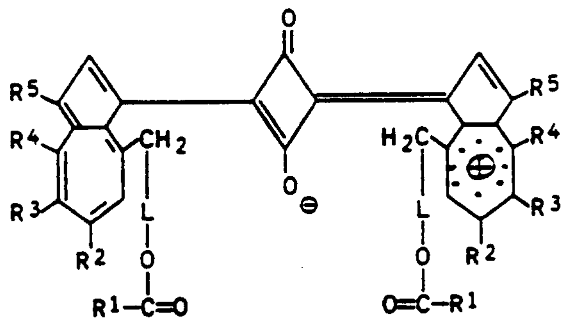

(I)

where

L is $C_1$-$C_{12}$-alkylene which may be substituted by phenyl,

$R^1$ is monounsaturated or polyunsaturated $C_2$-$C_{12}$-alkenyl, which may be phenyl-substituted, $C_5$-$C_7$-cycloalkyl, pyrrolyl, furanyl, thienyl or pyridyl and

$R^2$, $R^3$, $R^4$ and $R^5$ are identical or different and each is independently of the others hydrogen or $C_1$-$C_{12}$-alkyl which may be substituted by halogen, $C_1$-$C_{12}$-alkoxy, phenyl, substituted phenyl, $C_1$-$C_{12}$-alkoxycarbonyl or by cyano,

with the proviso that when $R^5$ is hydrogen the positions of the substituents $CH_2$-L-O-CO-$R^1$ and $R^4$ on either or both azulene rings may also be interchanged with each other within an azulene ring.

2. An azulenesquaric acid dye as claimed in claim 1, wherein $R^2$, $R^3$, $R^4$ and $R^5$ are each $C_1$-$C_6$-alkyl or hydrogen.

3. An azulenesquaric acid dye as claimed in claim 1, wherein $R^2$ and $R^4$ are each methyl and $R^3$ and $R^5$ are each hydrogen.

4. An azulenesquaric acid dye as claimed in claim 1, wherein $R^2$ and $R^4$ are each hydrogen, $R^3$ is isopropyl and $R^5$ is methyl.

5. An optical recording medium comprising a base material and a radiation-sensitive thin coating film which contains a dye with or without a binder, wherein the dye has the formula I

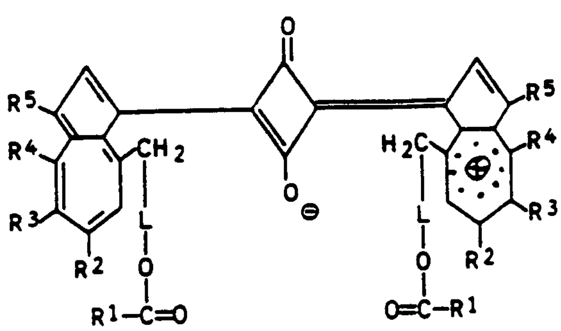

(I)

where

| | |
|---|---|
| L | is $C_1$-$C_{12}$-alkylene which may be substituted by phenyl, |
| $R^1$ | is monounsaturated or polyunsaturated $C_2$-$C_{12}$-alkenyl, which may be phenyl-substituted, $C_5$-$C_7$-cycloalkyl, pyrrolyl, furanyl, thienyl or pyridyl and |
| $R^2$, $R^3$, $R^4$ and $R^5$ | are identical or different and each is independently of the others hydrogen or $C_1$-$C_{12}$-alkyl which may be substituted by halogen, $C_1$-$C_{12}$-alkoxy, phenyl, substituted phenyl, $C_1$-$C_{12}$-alkoxycarbonyl or by cyano, |

with the proviso that when $R^5$ is hydrogen the positions of the substituents $CH_2$-L-O-CO-$R^1$ and $R^4$ on either or both azulene rings may also be interchanged with each other within an azulene ring.

6. An optical recording medium as claimed in claim 5, wherein $R^2$, $R^3$, $R^4$ and $R^5$ are each $C_1$-$C_6$-alkyl or hydrogen.

7. An optical recording medium as claimed in claim 5, wherein $R^2$ and $R^4$ are each methyl and $R^3$ and $R^5$ are each hydrogen.

8. An optical recording medium as claimed in claim 5, wherein $R^2$ and $R^4$ are each hydrogen, $R^3$ is isopropyl and $R^5$ is methyl.

9. A carbonyloxy-substituted azulene of the formula II

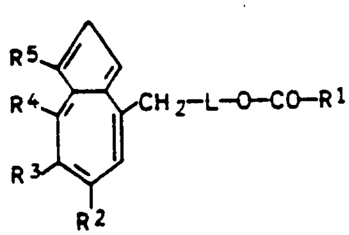

(II)

where

| | |
|---|---|
| L | is $C_1$-$C_{12}$-alkylene which may be substituted by phenyl, |
| $R^1$ | is monounsaturated or polyunsaturated $C_2$-$C_{12}$-alkenyl, which may be phenyl-substituted, $C_5$-$C_7$-cycloalkyl, pyrrolyl, furanyl, thienyl or pyridyl and |
| $R^2$, $R^3$, $R^4$ and $R^5$ | are identical or different and each is independently of the others hydrogen or $C_1$-$C_{12}$-alkyl which may be substituted by halogen, $C_1$-$C_{12}$-alkoxy, phenyl, substituted phenyl, $C_1$-$C_{12}$-alkoxycarbonyl or by cyano. |

## Revendications

1. Colorants d'acide azulène quadratique contenant des groupes esters de formule I

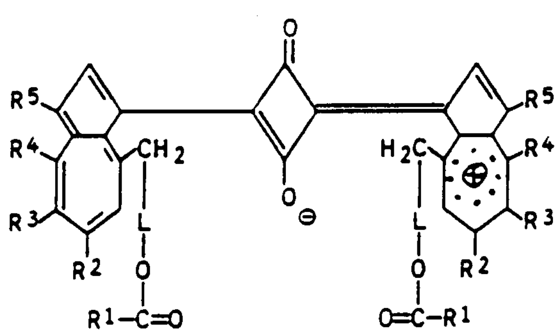

(I)

dans laquelle

| | |
|---|---|
| L | représente un groupe alkylene en $C_1$-$C_{12}$, qui est éventuellement substituer par un groupe phényle, |

$R^1$ represente un groupe alcenyle en $C_2$-$C_{12}$ à une ou plusieurs insaturations, qui peut être substitué par un groupe phényle, un groupe cycloalcényle en $C_5$-$C_7$, pyrrolyle, furanyle, thiényle au pyridyle et

$R_2$ $R^3$ $R^4$ et $R^5$ sont identiques ou différents et représentent chacun indépendamment l'un de l'autre un atome d'hydrogène ou un groupe alkyle en $C_1$-$C_{12}$, qui est éventuellement substitué par un atome d'halogène, un groupe alcoxy en $C_1$-$C_{12}$, un groupe phényle, phényle substitué, (alcoxy en $C_1$-$C_{12}$) carbonyle ou cyano,

à condition que lorsque $R^5$ représente un atome d'hydrogène, les positions sur le cycle des substituants $CH_2$-L-O-CO-$R^1$ et $R^4$ puissent également être échangées à l'intérieur d'un cycle azulène, sur l'un des cycles azulène ou sur les deux.

**2.** Colorants d'acide azulène quadratique selon la revendication 1, caractérisés en ce que $R^2$, $R^3$, $R^4$ et $R^5$ représentent chacun un groupe alkyle en $C_1$-$C_6$ ou un atome d'hydrogène.

**3.** Colorants d'acide azulène quadratique selon la revendication 1, caractérisés en ce que $R^2$ et $R^4$ représentent chacun un groupe méthyle et $R^3$ et $R^5$ représentent chacun un atome d'hydrogène.

**4.** Colorants d'acide azulène quadratique selon la revendication 1, caractérisés en ce que $R^2$ et $R^4$ représentent chacun un atome d'hydrogène, $R^3$ un groupe isopropyle et $R^5$ un groupe méthyle.

**5.** Support d'enregistrement optique, contenant un support ainsi qu'un film mince d'enduction sensible aux rayonnements et contenant un colorant et éventuellement un liant, caractérisé en ce que le colorant a la formule I

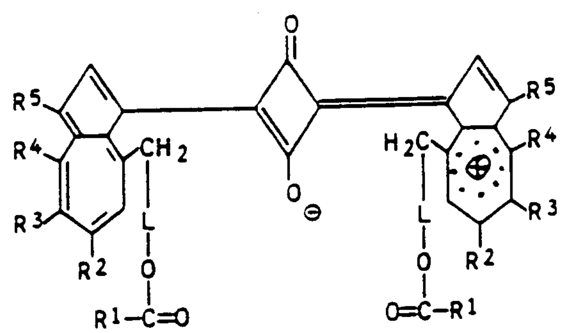

(I)

dans laquelle

L représente un groupe alkylène en $C_1$-$C_{12}$, qui est éventuellement substitué par un groupe phényle,

$R^1$ représente un groupe alcenyle en $C_2$-$C_{12}$ à une ou plusieurs insaturations, qui peut être substitué par un groupe phényle, un groupe cycloalcényle en $C_5$-$C_7$, pyrrolyle, furanyle, thiényle ou pyridyle et

$R^2$, $R^3$, $R^4$ et $R^5$ sont identiques ou différents et représentent chacun indépendamment l'un de l'autre un atome d'hydrogène ou un groupe alkyle en $C_1$-$C_{12}$, qui est éventuellement substitué par un atome d'halogène, un groupe alcoxy en $C_1$-$C_{12}$, un groupe phényle, phényle substitué, (alcoxy en $C_1$-$C_{12}$)carbonyle ou cyano,

à condition que lorsque $R^5$ représente un atome d'hydrogène, les positions sur le cycle des substituants $CH_2$-L-O-CO-$R^1$ et $R^4$ puissent également être échangés à l'intérieur d'un cycle azulène, sur l'un des cycles azulène ou sur les deux.

**6.** Support d'enregistrement optique selon la revendication 5, caractérisé en ce que $R^2$, $R^3$, $R^4$ et $R^5$ représentent chacun un groupe alkyle en $C_1$-$C_6$ ou un atome d'hydrogène.

**7.** Support d'enregistrement optique selon la revendication 5, caractérisé en ce que $R^2$ et $R^4$ représentent chacun un groupe méthyle et $R^3$ et $R^5$ représentent chacun un atome d'hydrogène.

**8.** Support d'enregistrement optique selon la revendication 5, caractérisé en ce que $R^2$ et $R^4$ représentent chacun un atone d'hydrogéne, $R^3$ un groupe isopropyle et $R^5$ un groupe méthyle.

**9.** Azulènes contenant des groupes esters de formule II

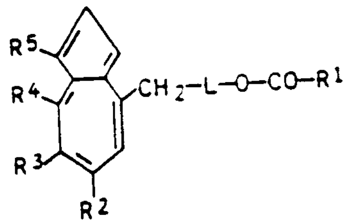

(II),

dans laquelle

L représente un groupe alkylene en $C_1$-$C_{12}$, qui est éventuellement substitué par un groupe phényle,

$R^1$ représente un groupe alcényle en $C_2$-$C_{12}$ à une ou plusieurs insaturations, qui peut être substitué par un groupe phényle, un groupe cyclonlcényle en $C_5$-$C_7$, pyrrolyle, furanyle, thiényle ou pyridyle,

$R^2$, $R^3$, $R^4$ et $R^5$ sont identiques ou différents et représentent chacun indépendnmment l'un de l'autre un atome d'hydrogène ou un groupe alkyle en $C_1$-$C_{12}$, qui est éventuellement substitué par un atome d'halogène, un groupe alcoxy en $C_1$-$C_{12}$, un groupe phényle, phényle substitué, (alcoxy en $C_1$-$C_{12}$)carbonyle ou cyano.